**Europäisches Patentamt**

**European Patent Office**

**Office européen des brevets**

(19)

(11) Publication number : **0 415 388 B1**

(12) # EUROPEAN PATENT SPECIFICATION

(45) Date of publication of patent specification :
**03.05.95 Bulletin 95/18**

(51) Int. Cl.⁶ : **G01N 27/327**, C12M 1/40,
G01N 27/38

(21) Application number : **90116573.8**

(22) Date of filing : **29.08.90**

(54) Method and apparatus for reviving an electrode of a biosensor.

(30) Priority : **30.08.89 JP 223614/89**

(43) Date of publication of application :
**06.03.91 Bulletin 91/10**

(45) Publication of the grant of the patent :
**03.05.95 Bulletin 95/18**

(84) Designated Contracting States :
**BE DE ES FR GB IT NL**

(56) References cited :
**DE-A- 3 822 911
US-A- 4 166 775
US-A- 4 772 375
US-A- 4 804 454
PATENT ABSTRACTS OF JAPAN vol. 7, no.
125 (P-200)(1270) 31 May 1983, & JP-A-58
041344**

(73) Proprietor : **DAIKIN INDUSTRIES, LIMITED
Umeda Center Building,
4-12 Nakazaki-nishi 2-chome,
Kita-ku
Osaka-shi, Osaka-fu 530 (JP)**

(72) Inventor : **Fujimura, Hidetaka
No. 2-103, 5-40,
Higashikusatsu 2-chome
Kusatsu-shi, Shiga 525 (JP)**
Inventor : **Inamoto, Tomoyuki
No. 2-303, 5-40, Higashikusatsu 2-chome
Kusatsu-shi, Shiga 525 (JP)**
Inventor : **Nagata, Yasuhiro
164-77, Kataoka-cho
Deceased (JP)**

(74) Representative : **Prüfer, Lutz H., Dipl.-Phys.
Harthauser Strasse 25d
D-81545 München (DE)**

EP 0 415 388 B1

## Description

The present invention relates to methods and apparatus for reviving an electrode of a biosensor, and more particularly to methods and apparatus for reviving an electrode of a biosensor by supplying a bias having a different polarity from the measurement bias polarity to the electrode before a measuring operation is started.

It is known that a physiologically active substance is capable of selectively detecting a very complicated organic compound, protein or the like with high sensitivity. With attention directed to this characteristic, research has been made in various biosensors.

A typical biosensor having an electrode unit and a physiologically active substance fixed thereon is proposed. The biosensor is used for detecting the existance of art object substance, the relative or active quantity of the object substance and the like based art an electrical signal output from the electrode unit corresponding to the biological reaction of the object substance, under the condition that a predetermined forward bias is applied to the electrode unit. For example, the electrode unit has a working electrode made of platinum and a counter electrode made of silver. An enzyme-immobilized membrane is fixed onto the electrode unit. When the object substance is to be measured, hydrogen peroxide is generated through an enzyme reaction of the object substance. Then the generated hydrogen peroxide reaches the surface of the electrode unit through a hydrogen peroxide penetration membrane. The electrode unit outputs an electrical signal corresponding to the quantity of hydrogen peroxide that reaches it. The existance of the object substance, the relative or active quantity of the object substance and the like are detected based on the electrical signal. Concretely, the working electrode has a forward bias of 0.6 volts applied with respect to the counter electrode.

In the biosensor described above, an interfering layer such as an oxidized layer and the like which interferes with electrical signals is generated on the working electrode, and the activity of the working electrode is lowered as an object substance measuring operation is continued with the forward bias being applied to the electrode unit. It is proposed that a reverse bias is accordingly applied to the electrode unit (the wording electrode has a reverse bias of about -0.6 volts applied with respect to the counter electrode, corresponding to the concrete example) after one or more measuring is performed, within the time period in which the measurment is not performed, so as to remove the interfering layer. Then the activity of the working electrode is revived. The level of the output signal from the electrode unit is raised to the original level. ( Refer to Japanese Patent Laid Open Sho 60-155959 ).

Lowered measuring sensitivity is recovered by applying the predetermined reverse bias voltage to the electrode unit within the time period in which the measurment is not performed, as is described above, then measuring with high sensitivity can be performed again.

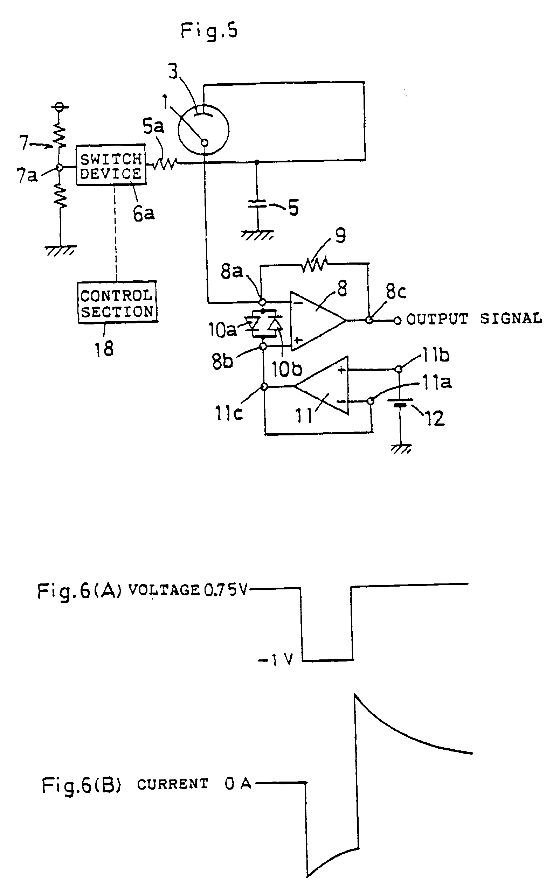

In the reviving apparatus described above, reverse bias for reviving is applied between the working electrode and the counter electrode ( refer to fig. 6(A)) every predetermined time (for example, every one hour) and just before starting of measurement. When the reverse bias for reviving is applied between the working electrode and the counter electrode, a large current flows in thin membranes fixed on a surface of an electrode unit (refer to fig. 6(B)). Thin membranes, especially a selective penetration membrane being positioned close to the electrode unit, are gradually destroyed, thereby the penetration quantity of substances generated by the reaction or substances consumed by the reaction increases. For example, when the biosensor is used to measure concentration of glucose, the hydrogen peroxide selective penetration membrane, made of a cellulose acetate membrane is destroyed, thereby the quantity of penetrating hygrogen peroxide increases. As a result, there is a disadvantage that an electrical signal provided from the electrode unit increases depending on number of times a reviving operation occurrs. Another disadvantage is that the life of the membrane or membranes is shortened by up to about one month. The life is a time period until a penetration ratio of the hydrogen peroxide selective penetration membrane increases by 10 %,

Such an apparatus is also known from DE-A-38 22 911 which also forms the generic portions of claim 1 and claim 5, respectively.

From the US-A-4 804 554 an oxygen concentration sensing apparatus is known which includes an oxygen pump element and a sensor cell element to be placed in a gas under measurement, and a device for generating a voltage value, and indicating a voltage to be generated across electrodes of the sensor cell element. The voltage to be applied to the sensor cell element is generated by an integration circuit which gradually increases the output voltage in accordance whith an integration time constant and becomes a stable when it reaches the output voltage of a voltage dividing circuit.

From the US-A-4 166 775 an electro chemical detection system is known for measuring the amount of carbon monoxide or similar oxidizable gases present in a gas phase mixtures wherein the gas phase mixture is brought into contact with a liquid electrolyte through a gas-permeable measuring electrode. The electrode is rejuvenated after each sample measurement by successively impressing an oxidizing (anodic) voltage, a reducing (cathodic) voltage and a working or reading voltage in an appropriate time sequence.

From JP-A-58-41344 a voltammetry analysis is known wherein a specific measurement voltage and a specific cleaning voltage is applied which are different. The resulting current is determined before a steady state is reached by curve fitting the current curve in a time current diagram.

It is an object of the present invention to suppress the degree of damage to the membrane or membranes fixed on a surface of an electrode unit.

This object is solved by a method according to claim 1.

The method first applies a predetermined reverse bias to a working electrode for a predetermined time period, then, applies a predetermined forward measurement bias to the working electrode for a predetermined time period, at least one of the reverse bias and the forward measurement bias being a bias voltage which gradually increases in absolute value up to a predetermined value.

It is preferred that the predetermined reverse bias is a reverse bias which is gradually increased until its absolute value reaches a predetermined value, and the forward measurement bias is a forward bias which is gradually increased until its absolute value reaches a predetermined value.

It is also preferred that an increasing ratio of the forward measurement bias is equal to an increasing ratio of the reverse bias.

It is further preferred that an increasing ratio of the forward measurement bias is greater than an increasing ratio of the reverse bias.

The object is also solved by an apparatus according to claim 5.

The apparatus comprises:

reviving voltage applying means for applying a reviving voltage to a working electrode;

measurement voltage applying means for applying a measurement voltage to the working electrode;

time constant means for controlling at least one of the voltages applied by the voltage applying means according to a predetermined time constant.

It is preferred that the time constant means controls the reviving voltage applied by the reviving voltage applying means and the measurement voltage applied by the measurement voltage applying means according to a predetermined time constant.

It is also preferred that the time constant means applies the same time constants to both voltages applied by both voltage applying means.

It is further preferred that the time constant means applies a longer time constant to the measurement voltage applied by the measurement voltage applying means than the time constant applied to the reviving voltage applied by the reviving voltage applying means.

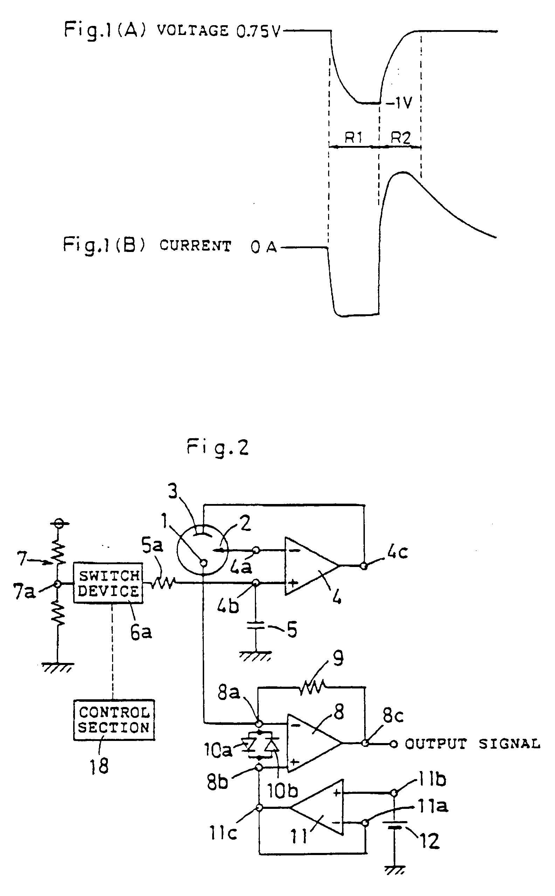

In this reviving method and apparatus, when an interfering layer on the working electrode which interferes with the turning on of electricity is reduced and activity of the working electrode is revived by applying the reverse bias to the working electrode, a reverse bias with gradually increasing absolute value is applied to the working electrode (refer to fig. 1(A)) instead of a reverse bias having a constant predetermined value as applied from the beginning of reviving. A current undershoot is remarkably suppressed, accordingly (refer to fig. 1(B)).

As a result, destruction of the membrane or membranes fixed to the surface or the electrode unit is remarkably suppressed, thereby to increase the level of an electrical output signal output from the electrode unit, depending on an increase in the number of times a reviving operation has occurred, is remarkably suppressed. Also, the life of the membrane or membranes is remarkably lengthened.

A current overshoot is also suppressed remarkably by applying a forward measurement bias with gradually increasing absolute value. As a result, destruction of the membrane or membranes fixed to the surface of the electrode unit is further remarkably suppressed.

More specifically, the inventors recognized that a large current flowing at the instant of applying a reverse bias for reviving caused the membrane or membranes to be destroyed. The inventors considered the cause of such a large amount of current, and found the cause is that the charging current into an electric double layer increases following sudden changing of the bias. Taking this knowledge into consideration, when the bias is gradually changed instead of suddenly changing it, a large amount of current is securely prevented from flowing, and the damage to the membrane or membranes is remarkably suppressed. In other words, the life of the membrane or membranes is remarkably lengthened.

These and other objectives, features and advantages of the present invention will be more readily understood upon consideration of the present invention, taken in conjunction with the accompanying drawings.

Figures 1(A) and 1(B) are diagrams schematically showing variations of bias voltage and current when the electrode unit is revived;

Figure 2 is an electronic circuit diagram of an electrode reviving apparatus in accordance with a first embodiment of the present invention;

Figure 3 is a vertical sectional view of the center portion of an electrode unit;

Figures 4 is a diagram showing actual variations of current when the electrode unit is revived;

Figure 5 is an electronic circuit diagram of an electrode reviving apparatus in accordance with a second embodiment of the present invention;

Figures 6(A) and 6(B) are diagrams showing variations of bias voltage and current when the electrode unit is revived in conventional manner.

Fig. 2 is an electronic circuit diagram of an electrode reviving apparatus in accordance with a first embodiment of the present invention.

The apparatus revives an electrode unit having three electrodes, such as a working electrode 1 made of platinum, a reference electrode 2 and a counter electrode 3, both made of silver.

The reference electrode 2 and the counter electrode 3 are connected to an inverting input terminal 4a and an output terminal 4c of an operational amplifier 4, respectively. A condenser 5, provided as a biasing source, is connected between a non-inverting input terminal 4b of the operational amplifier 4 and ground. A connecting point of the condenser 5 and the operational amplifier 4 is connected to a high voltage tap 7a of a voltage divider 7 having resistances, through a resistance 5a and a switch device 6a. That is, a tine constant circuit is provided by the resistance 5a and the condenser 5. An inverting input terminal 8a of a current/voltage converting operational amplifier 8 for providing measurement signals is connected to the working electrode 1. A current/voltage converting resistance 9 is connected between an output terminal 8c and the inverting input terminal, 8a of the current/voltage converting operational amplifier 8. Diodes 10a and 10b are connected in paralell and in reverse polarity with respect to one another between the inverting input terminal 8a and a non-inverting input terminal 8b of the current/voltage converting operational amplifier 8. A DC power source 12 for measuring is connected to a non-inverting input terminal 11b of a buffer amplifier 11. An output terminal 11c of the buffer amplifier 11 is connected directly to an inverting input terminal 11a of the buffer amplifier 11. The output terminal 11c of the buffer amplifier 11 is connected to the non-inverting input terminal 8b of the current/voltage converting operational amplifier 8. The DC power source 12 is used for applying a forward bias of 0.75 volts to the working electrode 1. The high voltage tap 7a is used for applying a bias of -1 volt to the wording electrode 1. A control section 18 is used to control the operation of the switch device 6a.

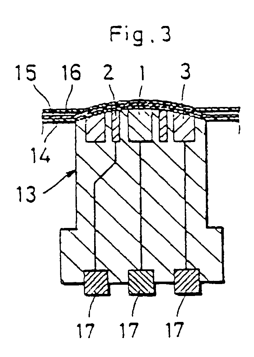

Fig. 3 is a vertical sectional view of the center portion of an electrode unit to which the reviving apparatus is applied.

The working electrode 1 is provided at a predetermined position in an electrode unit body 13. The ring shaped reference electrode 2 and the ring shaped counter electrode 3, both electrodes 2 and 3 surrounding the periphery of the working electrode 1, are provided in the electrode unit body 13 in this order. The electrode unit body 13 has a convex surface on the side on which the working electrode 1, the reference electrode 2 and the counter electrode 3 are provided. A hydrogen peroxide selective penetration membrane 14 made of cellulose acetate and the like, a glucose oxidaze immobilized membrane (hereinafter referred to as a GOD immobilized membrane ) 15 and a diffusion-limiting membrane 16 made of polyvinyl acetate and the like are provided in this order to fit the convex surface of the electrode unit body 13. Terminals 17 for providing output signals are provided in the electrode unit body 13, and the terminals 17 are connected to the working electrode 1, the reference electrode 2 and the counter electrode 3, respectively.

The operation of the sensor for measuring glucose concentrations having the arrangement described above is as follows.

When measuring of glucose concentration is carried out, the non-inverting input terminal 4b of the operational amplifier 4 is connected to the high voltage tap 7a of the voltage divider 7 by operating the switch device 6a through the resistance 5a. When the non-inverting input terminal 4b is connected to the high voltage tap 7a through the resistance 5a, the high voltage tap 7a is to apply a reverse bias of -1 volt between the working electrode 1 and the reference electrode 2. The actual voltage applied between the working electrode 1 and the reference electrode 2 is determined based on the voltage between the terminals of the condenser 5, thereby the reverse bias gradually increases by a time constant which is determined by the resistance 5a and the condenser 5, as is illustrated in fig. 1(A), region R1. The time constant has a greater value, for example 0.5 seconds, than a time constant, usually 0.001-0.1 seconds, of a CR circuit which is used for removing noise. In this condition, a current undershoot is scarcely recognized as is illustrated in fig. 1(B), region R1. Also, the activity of the electrode unit is sufficiently revived because the current has a sufficient value to reduce interfering substances on the surface of the electrode unit.

After the reverse biasing operation is performed for a petermined time period, the switch device 6a is operated to turn off. The DC power source 12 is to apply a forward bias of 0.75 volts between the working electrode 1 and the reference electrode 2. The actual voltage applied between the working electrode 1 and the reference electrode 2 gradually increases as is illustrated in fig. 1(A), region R2, because the voltage between the terminals of the condenser 5 gradually decreases according to the predetermined time constant. In this condition, a current overshoot is remarkably suppressed as is illustrated in fig. 1(B), region R2. Furthermore, the quantity of generated hydrogen, hydrogen ions and the like is lessened because a current undershoot is scarcely recognized, thereby a time period for consuming generated hydrogen, hydrogen ions and the like before it is possible to perform measuring with predetermined accuracy, is shortened. Also, accuracy of the measurement is improved because destruction of the membrane or membranes is remarkably reduced. Furthermore, the life of the membrane or membranes is extended up to about one year while the life of the membrane or membranes applied in a conventional reviving apparatus is about one month.

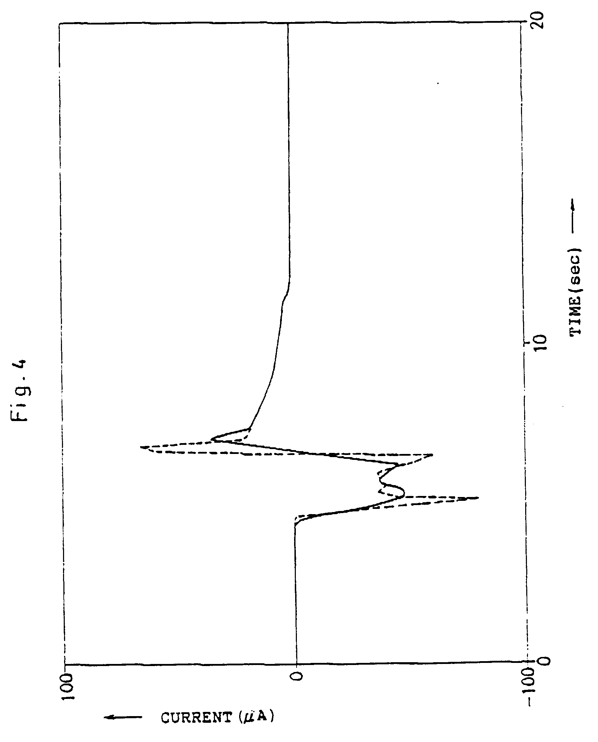

Fig. 4 is a diagram showing variations of currents, one current corresponding to the time constant of 0 seconds and another current corresponding to the time constant of 0.5 seconds. A broken line corresponds to the time constant of 0 seconds, and shows an undershoot of -78.9 micro amperes and an overshoot of 66.0 micro amperes. A solid line corresponds to the time constant of 0.5 seconds, and shows an undershoot of -48.0 micro amperes and an overshoot of 36.3 micro amperes. As is apparent from the drawing, the undershoot and overshoot are remarkably suppressed. In both cases, after the undershoot has occurred, first the current decreases in absolute value, then the current increases in absolute value. This variation of current may be caused in that the first undershoot is caused by flowing charging current, then the charging current decreases, thereafter, the surface of the electrode unit is completely charged and has a potential for generating hydrogen, thereby the current again increases in absolute value.

After reviving is finished, a signal corresponding to the concentration of glucose is output as follows by dropping an object solution onto the electrode unit.

The dropped object solution is guided to the GOD immobilized membrane 15 with limited penetration by the glucose to some degree, by the diffusion-limiting membrane 16. Then, the following reaction takes place:

$$\text{Glucose} + O_2 + H_2O \xrightarrow{\text{GOD}} \text{Gluconic acid} + H_2O_2$$

Hydrogen peroxide, the quantity of which corresponds to concentration of existing glucose, is accordingly generated. The generated hydrogen peroxide is guided to the surface of the working electrode which is revived to have sufficient activity, through the hydrogen peroxide selective penetration membrane 14. The forward bias is kept applied to the working electrode 1. An oxydation reaction is carried out on the surface of the working electrode 1 and current corresponding to the amount of hydrogen peroxide flows in through the working electrode 1. The current is applied to the inverting input terminal 8a of the current/voltage converting operational amplifier 8, then a voltage signal is output from the output terminal 8c of the current/voltage converting operational amplifier 8. The voltage signal is generated by adding an offset voltage caused by the forward bias and a voltage signal being proportional to the current.

Thereafter, only the voltage signal proportional to the current is extracted, then the extracted voltage signal is differentiated to obtain a first-order differential value, then a peak value of the first-order differential value is detected. Finally, a glucose concentration detection signal with high accuracy is obtained by performing the necessary operations.

Fig. 5 is an electronic circuit diagram of an electrode reviving apparatus in accordance with a second embodiment of the present invention.

Different points from the first embodiment are as follows,

(1) an electrode unit having two electrodes, one is the working electrode 1 made of platinum while another, the counter electrode 3 made of silver, is used for being revived, and

(2) the counter electrode 3 is directly connected to the switch device 6a by omitting the operational amplifier 4.

In this embodiment, undershoot and overshoot are remarkably reduced when the electrode unit is revived. Also, a time period between removal of interfering substances to when it is possible to start actual measuring can be shortened. Furthermore, the accuracy of measurement is improved and the life of the membrane or membranes are lengthened because the destruction of the membrane or membranes are remarkably suppressed.

This invention is not limited to the foregoing embodiments. Preferably, the voltage for reviving is changed. Preferably, the time period for applying the voltage is changed corresponding to the voltage value. Preferably, the reviving method and apparatus are applied to an apparatus for measuring concentrations of substances other than glucose. Preferably, a programmable power source for varying output voltage, the source being controlled by a microcomputer, is employed instead of the time constant circuit comprising the resistance 5a and the condenser 5.

The terms and expressions which have been employed here are used as terms of description and not of limitations, and there is no intention, in the use of such terms and expressions of excluding equivalents of the features shown and described, or portions thereof, it being recognized that various modifications are possible within the scope of the invention as claimed.

## Claims

1. A method for reviving an electrode unit of a biosenser which includes a working electrode (1) and a counter electrode (3),

   which measures a concentration of an object substance based upon an electrical signal generated between the working electrode (1) and the counter electrode (3) when a predetermined forward measurement bias is applied to the working electrode (1) on which a physiologically active substance is placed, the electrical signal being generated based upon a biological reaction of the object substance;

   which applies a predetermined reverse bias to the working electrode (1) for a predetermined time period; and

   which apply a predetermined forward measurement bias to the working electrode (1),

   that characterized in at least one of the biases is a bias which gradually increases in absolute value to reach a predetermined value.

2. A method as set forth in claim 1, wherein the predetermined reverse bias is a reverse bias which is gradually increased until its absolute value reaches a predetermined value, and the predetermined forward measurement bias is a forward bias which is gradually increased until its absolute value reaches a predetermined value.

3. A method as set forth in claim 2, wherein an increasing ratio of the forward measurement bias is equal to an increasing ratio of the reverse bias.

4. A method as set forth in claim 2, wherein an increasing ratio of the forward measurement bias is greater than an increasing ratio of the reverse bias.

5. An apparatus for reviving an electrode unit of a biosensor which includes:

   a working electrode (1) and a counter electrode (3),

   the apparatus measuring a concentration of an object substance based upon an electrical signal generated between said working electrode (1) and said counter electrode (3) when a predetermined forward measurement bias is applied to said working electrode (1) on which a physiologically active substance is placed, said electrical signal being generated based upon a biological reaction of said object substance;

   reviving voltage applying means (4, 6a, 7a) for applying a reviving voltage to said working electrode (1); and

   measurement voltage applying means (12) for applying a measurement voltage to said working electrode (1);

   characterized by:

   time constant means (5, 5a) for controlling at least one voltage applied by one of said voltage applying means (4, 6a, 7a, 12) according to a predetermined time constant.

6. An apparatus as set forth in claim 5, wherein said time constant means (5, 5a) controls the reviving voltage applied by said reviving voltage applying means (4, 6a, 7a) according to a first predetermined time constant, and controls the measurement voltage applied by said measurement voltage applying means (12) according to a second predetermined time constant.

7. An apparatus as set forth in claim 6, wherein said first and second time constants are the same time.

8. An apparatus as set forth in claim 6, wherein said second time constant is greater than said first time constant.

## Patentansprüche

1. Verfahren zum Wiederbeleben einer Elektrodeneinheit eines Biosensors, der eine Arbeitselektrode (1) und eine Gegenelektrode (3) enthält,

das eine Konzentration einer Objektsubstanz auf der Grundlage eines elektrischen Signales, das zwischen der Arbeitselektrode (1) und der Gegenelektrode (3) erzeugt wird, mißt, wenn eine vorbestimmte Vorwärtsmeßspannung an die Arbeitselektrode (1) angelegt wird, auf der eine physiologisch aktive Substanz angeordnet ist, wobei das elektrische Signal auf der Grundlage einer biologischen Reaktion der Objektsubstanz erzeugt wird;

das eine vorbestimmte negative Vorspannung an die Arbeitselektrode (1) während einer vorbestimmten Zeitdauer anlegt;

das eine vorbestimmte Vorwärtsmeßspannung an die Arbeitselektrode (1) anlegt;

dadurch gekennzeichnet, daß mindestens eine der Spannungen eine Spannung ist, die sich im Absolutwert allmählich zum Erreichen eines vorbestimmten Wertes erhöht.

2. Verfahren nach Anspruch 1, bei dem die vorbestimmte negative Vorspannung eine negative Vorspannung ist, die sich allmählich erhöht, bis ihr Absolutwert einen vorbestimmten Wert erreicht, und die vorbestimmte Vorwärtsmeßspannung eine Vorwärtsvorspannung ist, die sich allmählich erhöht, bis ihr Absolutwert einen vorbestimmten Wert erreicht.

3. Verfahren nach Anspruch 2, bei dem ein Erhöhungsverhältnis der Vorwärtsmeßspannung gleich einem Erhöhungsverhältnis der negativen Vorspannung ist.

4. Verfahren nach Anspruch 2, bei dem ein Erhöhungsverhältnis der Vorwärtsmeßspannung größer als ein Erhöhungsverhältnis der negativen Vorspannung ist.

5. Gerät zum Wiederbeleben einer Elektrodeneinheit eines Biosensors, das aufweist:

eine Arbeitselektrode (1) und eine Gegenelektrode (3),

wobei das Gerät eine Konzentration einer Objektsubstanz mißt auf der Grundlage eines elektrischen Signales, das zwischen der Arbeitselektrode (1) und der Gegenelektrode (3) erzeugt wird, wenn eine vorbestimmte Vorwärtsmeßspannung an die Arbeitselektrode (1) angelegt ist, auf der eine physiologisch aktive Substanz angeordnet ist, wobei das elektrische Signal auf der Grundlage einer biologischen Reaktion der Objektsubstanz erzeugt wird;

Wiederbelebungsspannungsanlegemittel (4, 6a, 7a) zum Anlegen einer Wiederbelebungsspannung an die Arbeitselektrode (1);

Meßspannungsanlegemittel (12) zum Anlegen einer Meßspannung an die Arbeitselektrode (1);

gekennzeichnet durch:

Zeitkonstantenmittel (5, 5a) zum Steuern von mindestens einer Spannung, die durch eines der Spannungsanlegemittel (4, 6a, 7a, 12) angelegt ist gemäß einer vorbestimmten Zeitkonstante.

6. Gerät Anspruch 5,

bei dem das Zeitkonstantenmittel (5, 5a) die durch das Wiederbelebungsspannungsanlegemittel (4, 6a, 7a) angelegte Wiederbelebungsspannung gemäß einer ersten vorbestimmten Zeitkonstante steuert und die durch das Meßspannungsanlegemittel (12) angelegte Meßspannung gemäß einer zweiten vorbestimmten Zeitkonstanten steuert.

7. Gerät nach Anspruch 6, bei dem die erste und zweite Zeitkonstante die gleichen sind.

8. Gerät nach Anspruch 6, bei dem die zweite Zeitkonstante größer als die erste Zeitkonstante ist.

## Revendications

1. Procédé pour réactiver un bloc d'électrodes d'un capteur biologique qui comprend une électrode de travail (1) et une contre-électrode (3),

qui mesure une concentration d'une substance objet en se basant sur un signal électrique généré entre l'électrode de travail (1) et la contre-électrode (3) lorsqu'une polarisation directe de mesure prédéterminée est appliquée à l'électrode de travail (1) sur laquelle une substance physiologiquement active est placée, le signal électrique étant généré en se basant sur une réaction biologique de la substance objet ;

qui applique une polarisation inverse prédéterminée à l'électrode de travail (1) pendant une période de temps prédéterminée ; et

qui applique une polarisation directe de mesure prédéterminée à l'électrode de travail (1),

caractérisé en ce qu'au moins une des polarisations est une polarisation dont la valeur absolue s'accroît graduellement pour atteindre une valeur prédéterminée.

2. Procédé selon la revendication 1, dans lequel la polarisation inverse prédéterminée est une polarisation inverse qui est augmentée graduellement jusqu'à ce que sa valeur absolue atteigne une valeur prédéterminée, et la polarisation directe de mesure prédéterminée est une polarisation directe qui est augmentée graduellement jusqu'à ce que sa valeur absolue atteigne une valeur prédéterminée.

3. Procédé selon la revendication 2, dans lequel un rapport croissant de la polarisation directe de mesure est égal à un rapport croissant de la polarisation inverse.

4. Procédé selon la revendication 2, dans lequel un rapport croissant de la polarisation directe de mesure est supérieur à un rapport croissant de la polarisation inverse.

5. Procédé pour réactiver un bloc d'électrodes d'un détecteur biologique qui comprend :

une électrode de travail (1) et une contre-électrode (3),

l'appareil mesurant la concentration d'une substance objet en se basant sur un signal électrique généré entre ladite électrode de travail (1) et ladite contre-électrode (3) quand une polarisation directe de mesure prédéterminée est appliquée à ladite électrode de travail (1) sur laquelle une substance physiologiquement active est placée, ledit signal électrique étant généré en se basant sur une réaction biologique de ladite substance objet ;

un moyen d'application d'une tension de réactivation (4, 6a, 7a) destiné à appliquer une tension de réactivation sur ladite électrode de travail (1) ; et

un moyen d'application d'une tension de mesure (12) destiné à appliquer une tension de mesure sur ladite électrode de travail (1) ;

caractérisé en ce que :

un moyen de constante de temps (5, 5a) commande au moins une tension appliquée par un desdits moyens d'application de tension (4, 6a, 7a, 12) en fonction d'une constante de temps prédéterminée.

6. Appareil selon la revendication 5, dans lequel ledit moyen de constante de temps (5, 5a) commande la tension de réactivation appliquée par ledit moyen d'application de la tension de réactivation (4, 6a, 7a) en fonction d'une première constante de temps prédéterminée, et commande la tension de mesure appliquée par ledit moyen d'application de la tension de mesure (12) en fonction d'une seconde constante de temps prédéterminée.

7. Appareil selon la revendication 6, dans lequel la première et la seconde constante de temps ont la même valeur.

8. Appareil selon la revendication 6, dans lequel ladite seconde constante de temps est supérieure à ladite première constante de temps.

Fig.1(A) VOLTAGE 0.75V

−1V

R1 R2

Fig.1(B) CURRENT 0 A

Fig.2

SWITCH DEVICE

CONTROL SECTION

OUTPUT SIGNAL

Fig. 3

Fig. 4

Fig.5

SWITCH
DEVICE

CONTROL
SECTION

OUTPUT SIGNAL

Fig.6(A) VOLTAGE 0.75V

−1 V

Fig.6(B) CURRENT 0 A